# FASCICULE DE BREVET EUROPEEN

(11) **EP 1 641 476 B1**
(45) Date de publication et mention de la délivrance du brevet: **29.03.2017**
(21) Numéro de dépôt: 04767603.6
(22) Date de dépôt: 07.07.2004
(51) Int. Cl.: A61K 31/19, A61K 31/05, A61K 31/235, A61K 31/375, A61K 31/7004, A61K 31/20, A61K 31/085, A61K 31/201, A61K 36/16, A61K 8/18, A61K 8/34, A61K 8/36, A61K 8/37, A61K 8/44, A61K 8/49, A61K 8/60, A61K 8/67, A61K 8/97, A61P 17/10

(54) **COMBINAISON D'ANTIOXYDANTS DANS UNE COMPOSITION A USAGE DERMATOLOGIQUE ET/OU COSMETIQUE**
KOMBINATION VON ANTIOXIDANTIEN IN EINER DERMATOLOGISCHEN UND/ODER KOSMETISCHEN ZUSAMMENSETZUNG
COMBINATION OF ANTIOXIDANTS IN A DERMATOLOGICAL AND/OR COSMETIC COMPOSITION

(30) Priorité: 07.07.2003 FR 0308288
(43) Date de publication de la demande: 05.04.2006
(73) Titulaire: THOREL, Jean-Noël, 75014 Paris (FR)
(72) Inventeur: THOREL, Jean-Noël, 75014 Paris (FR)
(74) Mandataire: Cabinet Laurent & Charras
(86) Numéro de dépôt international: PCT/FR2004/001768
(87) Numéro de publication internationale: WO 2005/004891

(56) Documents cités:
- EP-A- 1 077 066
- EP-A2- 0 676 187
- WO-A-99/22728
- WO-A1-02/11745
- CN-A- 1 186 690
- DE-A1- 4 328 871
- DE-A1- 10 034 328
- FR-A- 2 099 582
- GB-A- 1 476 717
- US-A1- 2003 190 338
- MORRIS G E: "Use of vitamin C in acne vulgaris." A. M. A. ARCHIVES OF DERMATOLOGY AND SYPHILOLOGY. SEP 1954, vol. 70, no. 3, septembre 1954 (1954-09), pages 363-364, XP008042755 ISSN: 0096-5979
- PATENT ABSTRACTS OF JAPAN vol. 0112, no. 90 (C-447), 18 septembre 1987 (1987-09-18) & JP 62 084021 A (SHISEIDO CO LTD), 17 avril 1987 (1987-04-17)
- KUBO I ET AL: "NON-ANTIBIOTIC ANTIBACTERIAL ACTIVITY OF DODECYL GALLATE", BIOORGANIC & MEDICINAL CHEMISTRY, PERGAMON, GB, vol. 11, no. 4, 1 February 2003 (2003-02-01), pages 573-580, XP001153507, ISSN: 0968-0896, DOI: 10.1016/S0968-0896(02)00436-4

## Description

La présente invention se rapporte au traitement de l'acné et des désordres cutanés liés à la formation de comédons.

Le comédon est la première lésion de l'acné vulgaire et cette lésion résulte de l'obstruction par des cellules de la paroi du follicule du canal empêchant le sébum produit par les sébocytes (cellules des glandes sébacées) d'atteindre la surface de la peau. Le mélange de sébum et de cellules forme un bouchon appelé comédon, et provoque dans le pore ainsi bouché la prolifération de bactéries qui vivent normalement sur la peau comme les *Propionibactérium acnes* et *granulosum* et des levures notamment *Malassezia furfur.* Ces bactéries possèdent la particularité de métaboliser les triglycérides du sébum en libérant des acides gras qui provoquent l'inflammation des tissus.

La dermite séborrhéique en résultant se traduit par une éruption de taches ou de plaques rouges, recouvertes de squames grasses jaunâtres, plus ou moins prurigineuses, prédominant dans les zones riches en glandes sébacées. A la face, la topographie des lésions est évocatrice : sillon entre le nez et les lèvres, racine des sourcils, cuir chevelu, ailes du nez, plis des pavillons, conques des oreilles, conduits auditifs externes. Au cuir chevelu, l'atteinte fréquente se traduit par un état pelliculaire plus ou moins séborrhéique. Sur le tronc, on remarque deux zones fréquentes chez l'homme : le sternum et la région entre les deux omoplates.

Le sébum est un produit de sécrétion gras, riche en acides gras et notamment en squalène un carbure aliphatique à 30 atomes de carbone, précurseur du cholestérol. Le sébum joue un rôle positif important notamment dans la protection de la peau, mais il a également été établi depuis 1969 (Cunliffe, W.J. et al, Lancet, I, 685, 1969, The pathogenesis of acne) qu'il existe une corrélation entre le taux de sécrétion de sébum et la sévérité de l'acné.

Ainsi, dans la dermatologie et la cosmétique moderne, de nombreuses recherches sont menées pour mettre au point des compositions afin de réduire et de contrôler les sécrétions excessives des glandes sébacées, notamment afin de réduire les conséquences à la fois inesthétiques comme l'aspect gras et huileux de la peau et du cuir chevelu, mais également afin de réduire la formation des comédons et des inflammations résultantes.

Ces compositions ont des effets secondaires non négligeables comme la sécheresse cutanée, les sensations de tiraillement voire des inflammations dues à l'élimination du sébum qui ne peut plus jouer son rôle de protecteur.

S'agissant des traitements pour les cheveux, on citera les compositions capillaires décrites dans FR2099582, renfermant en solution au moins un dérivé phénolique, lesdites compositions étant destinées à désodoriser les cheveux, mais qui en plus de cette action désodorisante ralentissent les sécrétions de sébum, et dont l'application semble avoir pour résultat, soit un fort ralentissement des sécrétions, soit leur modification dans le sens d'une moins grande fluidité.

Le document CN 1 186 690 décrit une pommade comprenant un extrait de Ginkgo biloba riche en flavonoïdes pour le traitement de l'acné.

Le document KUBO et al. (Bioorg. & Med. Chem., 11 (4), 2003, pp 573-80) rapporte l'activité antibactérienne du dodécyl gallate contre *Proponibacteriulm acnes.*

Le document EP 1 077 066 décrit l'utilisation d'un dérivé de la vitamine C, éventuellement en combinaison avec le propyl gallate, pour le traitement de l'acné.

Le document GB 1 476 717 décrit un gel pour le traitement de l'acné comprenant un antioxydant dont par exemple le propyl gallate.

Des travaux scientifiques récents ont montrés que l'un des constituants du sébum, le squalène et notamment les produits d'oxydation du squalène avaient des propriétés comédogènes et également irritantes (Saint - Léger et al., British J. of Dermatology, 114, 543-552, 1986, Chiba K. et al, The J. of Toxicological Sciences, 25, 77-83, 2000 et Uchino, T. et al, Biol. Pharm. Bulletin 25(5), 605-610, 2002).

Plus précisément il a été établi que la composition du sébum des patients atteints d'acné est fortement enrichi en squalène.

La demanderesse a également montré que de l'oxydation des lipides constituant le sébum, résultait une augmentation de la viscosité dudit sébum, et une augmentation de la formation de comédons, le sébum moins fluide ne pouvant plus s'écouler des canaux folliculaires.
Cette oxydation étant potentiellement provoquée par les composants bactériens et les U.V., entraînant la formation de polymères visqueux qui épaississent le sébum et qui sont très comédogènes.

La présente invention concerne donc des compositions qui améliorent la qualité du sébum en évitant son épaississement et réduisent ainsi l'induction de la prolifération kératinocytaire, entraînant une diminution de la comédogènèse.

De façon surprenante les résultats sur les peaux acnéiques sont obtenus sans modification du flux de sébum, c'est-à-dire sans action sur la quantité de sébum produite mais en réduisant sa comédogénicité, en corrigeant sa qualité et en évitant son épaississement.

Elle concerne plus précisément des compositions permettant de maintenir la fluidité du sébum et plus particulièrement à une composition à usage dermatologique et/ou cosmétique, destinée au traitement de l'acné, des dermites séborrhéiques et des désordres cutanés liés à la formation de comédons, caractérisée en ce qu'elle comprend à titre de principe actif au moins des gallates, des flavonoïdes et du mannitol.

Plus précisément, l'invention concerne une composition dans laquelle l'antioxydant lipophile est le dodécyl gallate et les flavonoïdes sont apportés par des extraits de Ginkgo biloba.

La quantité de principe actif, c'est-à-dire d'antioxydant est comprise entre 0,0001 et 20 % en poids de la composition, et de préférence entre 0,0001 et 25 % en poids de la composition.

Préférentiellement, elle est comprise entre 0,001 et 2 % en poids de la composition.

L'invention décrit également un procédé de fluidification du sébum, caractérisé en ce qu'il comporte l'application sur la peau ou le cuir chevelu d'une composition selon l'invention, telle que précédemment définie.

Cette composition comprend dans un milieu pharmacologiquement acceptable les principes actifs selon l'invention.

Le milieu pharmacologiquement acceptable, c'est-à-dire l'environnement galénique doit être ni oxydable, ni oxydant, c'est-à-dire que Les excipients utilisés doivent être non oxydables et stables dans les conditions d'application sur la peau, ni provoquer une oxydation.

L'invention concerne donc une composition telle que précédemment définie caractérisée en ce qu'elle ne contient pas d'excipients susceptibles de s'oxyder dans les conditions d'utilisation.

Les compositions selon l'invention peuvent éventuellement contenir divers additifs non oxydables, tels que des agents de mise en suspension, des émulsifiants, des polymères anioniques, cationiques, non-ioniques, amphotères, des protéines, des vitamines, des tensioactifs, des huiles minérales ou végétales, des cires, des gommes et/ou des résines de silicone, des agents épaississants, des agents acidifiants ou alcalinisants, des solvants, des stabilisateurs de pH, des agents anti-UV, des conservateurs, des antibactériens et des antifongiques, des parfums ou autres adjuvants habituellement utilisés en cosmétique ou en dermatologie.

Préférentiellement, les compositions selon la présente invention se présentent sous une forme adaptée à l'administration par voie topique cutanée, et couvrent toutes les formes cosmétiques ou dermatologiques. Ces compositions contiennent un milieu cosmétiquement et/ou dermatologiquement acceptable, c'est-à-dire compatible avec la peau, les poils ou les cheveux. Ces compositions peuvent notamment être sous forme de crèmes, émulsions H/E, E/H ou émulsions multiples, solutions, suspensions, gels, laits, lotion, sticks ou encore poudres, adaptés à une application sur la peau, les lèvres et/ou les cheveux.

L'invention concerne également l'utilisation d'une composition dermatologique et/ou cosmétique selon l'invention pour le traitement de l'acné et ou des désordres cutané dus à la formation de comédons.

Elle concerne plus particulièrement l'utilisation telle que définie précédemment, caractérisée en ce que la composition comprend des gallates et des flavonoïdes.

Plus précisément, l'invention concerne ladite utilisation lorsque le gallate est le dodécyl gallate et
les flavonoïdes sont apportés par des extraits de Ginkgo biloba.

Elle concerne une utilisation telle que définie précédem ment, caractérisée en ce que la composition comprend en outre du mannitol.

Selon l'invention la quantité d'antioxydant utilisée est comprise entre 0,0001 et 20 % en poids de la composition, et de préférence entre 0,0001 et 10 % en poids de la composition.

Préférentiellement, elle est comprise entre 0,001 et 2 % en poids de la composition.

D'autres avantages et caractéristiques de l'invention apparaîtront mieux à la lecture des exemples donnés à titre d'exemples

### EXEMPLE 1 - PREPARATION D'UN MELANGE ACTIF (exemple de référence)

| | |
|---|---|
| Sepigel 305 (Polyacrylamide and C13-14 Isoparaffin and Laureth | 2.00 % |
| Dodécyl gallate | 0.0001 % |
| Mannitol | 0.50 % |
| Conservateur (Parabens) | 0.20 % |
| EDTA (séquestrant) | 0.10 % |
| Eau qsp | 100% |

### EXEMPLE 2 - EFFET D'UNE COMPOSITION SELON L'INVENTION

Une composition selon l'invention contenant à titre de principe actif du dodécyl gallate, du mannitol et un extrait de Ginkgo biloba a été testée sur un groupe d'une trentaine de volontaires.

Les sujets, âgés en moyenne de 23 ans (18 à 34 ans) présentent une acné juvénile polymorphe d'intensité moyenne c'est-à-dire présentant environ 40 lésions rétentionnelles ou inflammatoires en moyenne.

Seules les lésions rétentionnelles c'est-à-dire les microkystes et les comédons sont observés, par comptage sur tout le visage sauf la pyramide nasale. La composition selon l'invention a été appliquée de façon biquotidienne pendant 8 semaines sur le visage.

On note après 8 semaines d'application une diminution des lésions rétentionnelles pour au moins 40 % des sujets, avec 20% des sujets présentant une diminution de 50 à 90 % des microkystes, 15 % une diminution de 50 à 90 % des comédons et 10 % une disparition complète des comédons.

### EXEMPLE 3 - PREPARATION DE COMPOSITIONS

### (exemple de référence)

Ces compositions ont été obtenues par simple mélange des différents composants. Les quantités indiquées sont données en pourcentage en poids.

**Emulsion huile dans eau**

| | |
|---|---|
| Montanov 68 (Cetearyl Alcohol and Cetearyl Glucoside | 5.00 % |
| Huile de Jojoba | 5.00 % |
| BHT | 0.05 % |
| Isopropyl Palmitate | 7.00 % |
| Glycérine | 5.00 % |
| Allantoïne | 0.10 % |
| Mannitol | 3.00 % |
| Sepigel 305 (Polyacrylamide and C13-14 isoparaffin and Laureth-7) | 0.30 % |
| Phenonip | 0.50 % |
| Parfum | 0.50 % |
| Eau qsp | 100 % |

**Gel**

| | |
|---|---|
| Carbopol Ultrez 10 (sol. A 2 %) | 25.00 % |
| Triéthanolamine | 0.50 % |
| Mannitol | 2.00 % |
| Dodécyl gallate | 0.0001 % |
| Conservateur | 0.20 % |
| EDTA (séquestrant) | 0.10 % |
| Parfum | 0.50 % |
| Eau qsp | 100 % |

**Lotion**

| | |
|---|---|
| Mono Propylène Glycol | 1.00 % |
| Allantoïne | 0.30 % |
| Glycérine | 1.00 % |
| Cetiol HE (PEG-7 Glyceryl Cocoate) | 1.00 % |
| Azéilate de lysine | 5.00 % |
| BHA | 0.01 % |
| Conservateur | 0.20 % |
| Parfum | 0.50 % |
| Eau qsp | 100 % |

**Gel moussant peaux grasses séborrhéiques**

| | |
|---|---|
| Composé (Mannitol 90% et ascorbyl palmitate 10%) | 2,00 % |
| Parfum | 0,30 % |
| Chlorure de sodium | 1,00 % |
| Acide glycolique dans l'eau à 57 % | 0,50 % |
| Copolymère alcool de suif hydrogéné oxyéthyléné (60 OE) / myristyl glycol | 0, 90 % |
| Glycérine | 3,00 % |
| N-carboxyéthoxyéthyl N-cocoylamidoéthyl aminoacétate N-di-sodique dans l'eau à 38 % | 5,00 % |
| Lauryl éther sulfate de sodium (C12-14 70/30) (2,2 OE) dans l'eau à 28 % | 14,30 % |
| Di-éthanolamide d'acides gras de coprah | 0,70 % |
| Mélange d'alcool butylique oxyéthyléné (26 OE) oxypropyléné (26 OP), huile de ricin hydrogénée oxyéthylénée (40 OE) dans l'eau | 1,00 % |
| Eau déminéralisée | qsq 100 |

**Gel traitant peaux séborrhéiques**

| | |
|---|---|
| Palmitate d'ascorbyle | 1,00 % |
| Parfum | 0,20 % |
| Gomme de xanthane | 1,00 % |
| Glycérine | 2,00 % |
| Alcool éthylique | 20,00 % |
| Rutine | 0.10 % |
| Mélange d'alcool butylique oxyéthyléné (26 OE) oxypropyléné (26 OP), huile de ricin hydrogénée oxyéthylénée (40 OE) dans l'eau | 1,00 % |
| Eau déminéralisée | qsp 100 |

**Lotion purifiante peaux acnéïques et états pelliculaires**

| | |
|---|---|
| BHA | 0,05 % |
| Propyl gallate | 0,0005 % |
| Parfum | 0,20 % |
| Alcool éthylique | 20,00 % |
| Glycérine | 2,00 % |
| Mélange d'alcool butylique oxyéthyléné (26 OE) oxypropylène (26 OP), huile de ricin hydrogénée (40 OE) dans l'eau | 1,00 % |
| Octopirox | 0.20 % |
| Eau déminéralisée | qsp 100 |

Pour compléter l'action des compositions selon l'invention et en renforcer l'efficacité et la tolérance, d'autres actifs peuvent être associés aux antioxydants. Il en résulte des compositions dont les différenetes composantes de l'action permettent de répondre aux besoins complexes des peaux à tendance acnéique.

Les compositions selon l'invention peuvent en outre comporter des actifs ayant une activité kératolytique chois parmi les esters d'alpha-hydoxy acideset ou/ou l'acide salicylique qui permettent d'éliminer et de prévenir la formation des amas dde cornéocytes pouvant également favoriser la formation de comédons.

Elles peuvent également en outre comprendre un sel de zinc, par exemple le gluconate de zinc ayant une action séborégulatrice, par son action inhbitrice de la 5-alpha-réductase et à forte concentration une action bactéricide sur *Propionibacterium acnes,* dont la prolifération au sein du comédon est caractéristique de l'acné.

L'addition dans les compositions selon l'invetion de forte concentration d'un actif comprenant du zinc, permet de compléter l'action de traitements antibiotiques comme les traitements à l'érythromycine en applications locales ou par voie systémique.

Les compositions selon l'invention peuvent également comprendre un actif antiinflammatoire ou apaisant comme l'acide 18-beta-glycyrrhétique (enoxolone) dont le rôle antiinflammatoire endogène serait du à l'inhibition de l'enzyme responsable de la transformation du cortisol en cortisone ou un extrait de *Ginkgo biloba* décrit comme inhibiteur de la cascade inflammatoire.

L'acton de ces actifs est complétée par une base galénique comportant de la glycérine et du xylitol choisi pour leurs propriétés hydratantes

## Revendications

1. Composition à usage dermatologique et/ou cosmétique, destinée au traitement de l'acné, des dermites séborrhéiques et des désordres cutanés liés à la formation de comédons comprenant les antioxydants suivants : des flavonoïdes apportés par un extrait de Ginkgo biloba, du dodécyl gallate et du mannitol.

2. Composition selon la revendication 1, ***caractérisée* en ce que** la quantité d'antioxydants est comprise entre 0,0001 et 20 % en poids de la composition, et de préférence entre 0,0001 et 10 % en poids de la composition.

3. Composition selon la revendication 2, ***caractérisée* en ce que** la quantité d'antioxydants est comprise entre 0,001 et 2 % en poids de la composition.

4. Composition selon l'une des revendications 1 à 3 pour son utilisation dans le traitement de l'acné et/ou des dermites séborrhéiques.

## Patentansprüche

1. Zusammensetzung für dermatologische und/ oder kosmetische Zwecke zur Behandlung von Akne, seborrhoischer Dermatitis und Hautproblemen in Zusammenhang mit der Bildung von Komedonen, die die folgenden Antioxidantien enthält: Flavonoide aus einem Ginkgo biloba-Extrakt, Dodecyl-Gallat und Mannitol.

2. Zusammensetzung gemäß Anspruch 1, ***dadurch gekennzeichnet, dass*** die Menge an Antioxidantien zwischen 0,0001 und 20 Gewichts-% der Zusammensetzung, noch besser zwischen 0,0001 und 10 Gewichts-% der Zusammensetzung liegt.

3. Zusammensetzung gemäß Anspruch 2, ***dadurch gekennzeichnet, dass*** die Menge an Antioxidantien zwischen 0,001 und 2 Gewichts-% der Zusammensetzung liegt.

4. Zusammensetzung gemäß einem der Ansprüche 1 bis 3 zur Verwendung bei der Behandlung von Akne und/oder seborrhoischer Dermatitis.

## Claims

1. A composition for dermatological and/or cosmetic use intended for the treatment of acne, seborrheic dermatitis and skin disorders related to the formation of comedos, comprising the following antioxidants: flavonoids extracted from Gingko biloba, dodecyl gallate and mannitol.

2. The composition as claimed in claim 1, ***characterized* in that** the amount of antioxidants is between 0.0001 and 20% by weight of the composition, and preferably between 0.0001 and 10% by weight of the composition.

3. The composition as claimed in claim 2, ***characterized* in that** the amount of antioxidants is between 0.001 and 2% by weight of the composition.

4. The composition as claimed in claims 1 to 3 for use in the treatment of acne and/or seborrheic dermatitis.
